# EUROPÄISCHE PATENTANMELDUNG

(11) **EP 0 714 678 A1**
(43) Veröffentlichungstag der Anmeldung: **05.06.1996**
(21) Anmeldenummer: 94118636.3
(22) Anmeldetag: 26.11.1994
(51) Int. Cl.: A61N 5/06

(54) **Verfahren zur hautschonenden Emission**

(71) Anmelder: Imab Stiftung, FL-9496 Balzers (LI)
(72) Erfinder: Brück, Gernot, D-50858 Köln (DE)
(74) Vertreter: Stachow, E.-W., Prof. Dr.

(57) **Zusammenfassung**

Um eine hautschonende Bräunung zu erreichen, wird eine aus einem Strahler und einem Filter bestehende Emissionsquelle vorgeschlagen, die keine oder nur eine geringe langwellige UVA-Strahlung emittiert.

## Beschreibung

### Stand der Technik

Bei herkömmlichen Besonnungsanlagen werden im Hochdruckbereich Kombinationen aus Strahler, Reflektor und Filter eingesetzt.
Da eine geraume Zeit von der nicht richtigen Voraussetzung ausgegangen wurde, daß durch ein Übermaß an UVA-Strahlung eine der natürlichen Besonnung ähnliche Bräunung erzielt würde, wurden nur Strahler mit hohem UVA-Output eingesetzt. Um in Hg-Hochdruckstrahlern entsprechende Mengen von UVA bereitzustellen, wurden diese mit Eisen oder/und Gallium dotiert.
Zur Zeit werden für Bräunungsanlagen weltweit ausschließlich eisendotierte Hg-Hochdrucklampen eingesetzt.

### Problem

Aufgrund einer neuen Filtertechnik, mittels welcher sehr genau dosiert die Hg-Linie bei 313 nm zur Bräunung eingesetzt werden kann, entfällt die Forderung nach Übermengen von UVA-Licht. Außerdem verursachte dieses Übermaß an UVA nur eine kurzfristige grau-braune Färbung der Haut, die in keinem Fall mit einer Urlaubsbräune konkurrieren konnte.

Noch wesentlicher ist aber die Erkenntnis, daß gerade das langwellige UVA-Licht für die vorzeitige Hautalterung verantwortlich ist.

Sind aber die Hg-Hochdruckstrahler mit Eisen dotiert, so werden eine Vielzahl von UVA-Linien emittiert, und zwar mit einer recht dichten Besetzung zwischen 340 und 410 nm.

Aufgrund der übermäßigen UVA-Abstrahlung im vorgenannten Bereich, aber hauptsächlich im Bereich nach 365 nm, ergibt sich demzufolge auch eine starke Beanspruchung der Haut.

### Problemlösung

Da bekannt ist, daß insbesondere das UVA-Licht oberhalb von 365 nm als langwelliges UVA verantwortlich ist für die Hautalterung, sollte dieses Licht nicht oder nur sehr stark gedämpft auf den Solarienbesucher scheinen.

Es muß also Forderung werden, daß sich die Bestrahlung auf der Sonnenbank auf den Bereich zwischen 310 nm und 365 nm beschränkt.

Demzufolge müssen die Lichtquellen oder die Lichtsysteme entsprechend eingerichtet werden.

### Die Strahler-Filter-Kombination mit hautschonender Emission

Im Hochdruckbereich werden nur Kombinationen eingesetzt, wobei die Strahler und die Filter für die Emissionen verantwortlich sind.

Ist der Transmissionsbereich der Filter derart, daß durch die aufgehende Flanke die 313 nm Hg-Linie eingestellt werden kann und das sichtbare Licht nebst nahem IR geblockt wird, dann sind hierdurch die Möglichkeiten des gefärbten Glasfilters bereits erschöpft.

Um die Blockierung des langwelligen UVA's zu erreichen, müssen entweder Schichtfilter eingesetzt werden oder Strahler ohne Emissionslinien im UV oberhalb von 365 nm.

Bei dem erfindungsgemäßen Strahler handelt es sich um den bereits bekannten, undotierten Hg-Strahler, dessen Emission mittels der Filterscheibe auf nur zwei Hauptlinien reduziert wird. So wirkt die durch die Öffnungsflanke des Filters einstellbare 313 nm Linie für die Langzeitpigmentierung, wogegen die nahezu ungedämpfte 365 nm Linie die Direktpigmentierung der Haut verursacht. Längerwelliges UVA-Licht wird nicht erzeugt, so daß die Hautalterung aufgrund langwelligem UVA stark eingeschränkt bleibt.

Nach vorliegenden Veröffentlichungen liegt das Maximum der Wirkkurve für die Hautalterung bei 380 nm und beginnt bei ca. 360 nm. Aufgrund des Kurvenverlaufs geht die 365 nm Linie nur mit maximal 20 % in das Ergebnis ein, womit diese Emission um den Faktor 5 weniger zur Hautalterung beiträgt als längerwelliges UVA zwischen 380 und 400 nm.

Eine weitere Optimierung des Strahlers wird dadurch erreicht, daß durch entsprechende Dotierung der Bereich zwischen 313 nm und 365 nm mit Linien gefüllt wird, ohne daß dabei Linien im UVA nach 365 nm entstehen.
Vorzugsweise sollten die Linien zu Lasten der 365 nm Hg-Linie gehen, damit sich auf diese Weise eine Verschiebung der Strahlung zum kurzwelligen UVA ergibt.

### Beschreibung

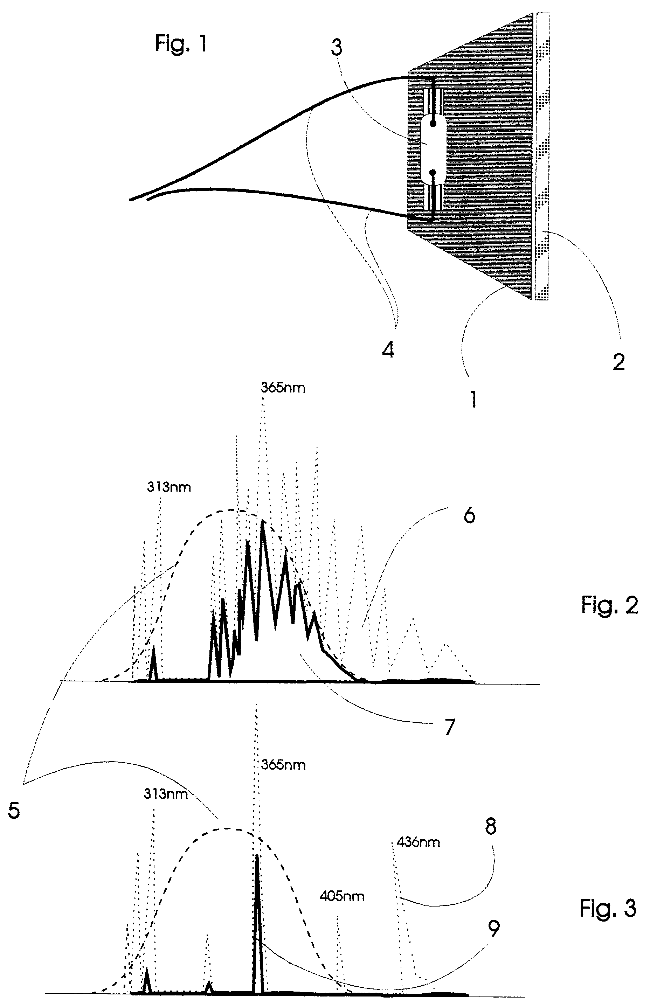

Anhand der Figuren 1, 2 und 3 wird das erfindungsgemäße Verfahren beschrieben.

In den Figuren 1 bis 3 werden dabei die folgenden Einzelheiten dargestellt:

In einem Reflektor 1 ist der Strahler 3 angebracht. Die notwendige Betriebsspannung wird über die Anschlüsse 4 bereitgestellt.
Vor dem Reflektor ist die Filterscheibe 2 angebracht, deren Transmissionskurve in den Figuren 2 und 3 als gestrichelte Linie 5 dargestellt ist.

Bei den herkömmlichen, mit Eisen dotierten Strahlern wird ein breites Emissionsband 6 abgestrahlt. Die Emission 7 nach dem Durchgang durch den Filter 2 weist noch große Mengen von langwelligem UVA auf.

Wird dagegen entsprechend dem erfindungsgemäßen Verfahren nur ein einfacher Hg-Hochdruckstrahler ohne Dotierung mit der Emissionskurve 8 eingesetzt, dann reduziert sich das langwellige UVA ganz erheblich, und es verbleibt die Emission 9, die hauptsächlich aus den Linien 313 nm und 365 nm besteht, sowie noch einen geringen Anteil der schwachen Linie 334 nm enthält.

## Patentansprüche

1. Verfahren für eine hautschonende Emission, **dadurch gekennzeichnet**, daß die Emission einer Lichtquelle für den Einsatz in Bräunungsanlagen keine oder reduzierte Anteile von langwelligem UVA aufweist.

2. Verfahren nach Anspruch 1, **dadurch gekennzeichnet**, daß es sich bei der Lichtquelle um eine Strahler-Filter-Kombination handelt.

3. Verfahren nach einem der Ansprüche 1 und 2, **dadurch gekennzeichnet**, daß es sich bei dem eingesetzten Strahler um eine undotierte Hg-Hochdrucklampe handelt.

4. Verfahren nach einem der Ansprüche 1 bis 3, **dadurch gekennzeichnet**, daß der Strahler so dotiert ist, daß keine wesentlichen Linien zwischen 365 und 436 nm entstehen, wohl aber der Bereich zwischen 313 nm und 365 vermehrt mit Linien besetzt wird.

5. Verfahren nach Anspruch 4, **dadurch gekennzeichnet**, daß die höhere Zahl an Emissionslinien zu Lasten der 365 nm Linien geht.

6. Verfahren nach einem der Ansprüche 1 bis 5, **dadurch gekennzeichnet**, daß dann, wenn eine größere Anzahl von Linien zwischen 313 nm und 365 nm zur Verfügung stehen, durch den Filter alle Linien über 360 nm absorbiert werden.
